# EUROPÄISCHE PATENTANMELDUNG

(11) **EP 0 826 348 A2**
(43) Veröffentlichungstag der Anmeldung: **04.03.1998**
(21) Anmeldenummer: 97111519.1
(22) Anmeldetag: 08.07.1997
(51) Int. Cl.: A61F 13/02

(54) **Antimikrobielle Wundauflagen**

(30) Priorität: 06.08.1996 DE 19631421
(71) Anmelder: Beiersdorf Aktiengesellschaft, 20245 Hamburg (DE)
(72) Erfinder: Knieler, Roland, Dr., 22529 Hamburg (DE); v. Wolff, Axel, 21447 Handorf (DE)

(57) **Zusammenfassung**

Wundauflage, die dadurch gekennzeichnet ist, daß sie aus einem hydrophoben, bakterienadsorbierenden Material besteht, das einen antimikrobiellen Wirkstoff enthält, der nicht in die Wunde abgegeben wird. Bevorzugt besteht sie aus einem Gemisch von hydrophoben Fasern und den antimikrobiellen Wirkstoff enthaltenden Fasern.

## Beschreibung

Die Erfindung betrifft Wundauflagen, die zur Behandlung infizierter Wunden oder zum vorbeugenden Schutz vor Wundinfektionen eingesetzt werden können.

Die antimikrobielle Behandlung von Wunden oder die vorbeugende Behandlung von Wunden zum Schutz vor Infektionen durch Mikroorganismen ist seit langem bekannt. Beispielhaft sei hier die Anwendung von Oxidantien (z.B. Jodtinktur) oder Antiseptika (z.B. Salben mit Silbersulfadiazin) genannt. Wenn Oxidantien oder andere antimikrobielle Wirkstoffe in Reinstform, in Lösung, als Salbe oder in einer anderen Darreichungsform eingesetzt werden, ist der Wirkungsmechanismus immer vergleichbar. Mikroorganismen wie Bakterien werden zwar sicher abgetötet. Es bleiben jedoch eine Reihe von Nachteilen zu nennen:
Abgestorbene Bakterien verbleiben in der Wunde.
Wunden können nach der Applikation nicht sicher vom Wirkstoff gereinigt werden, da er sich in der ganzen Wunde verteilt.
Und schließlich können diese Wirkstoffe, wenn sie sich frei in der Wunde befinden, auch Zellen und Substanzen, die die Wundheilung fördern, in der Wundflüssigkeit angreifen und zerstören (z.B. Schädigung heilungsfördernder Eiweißstoffe durch Oxidationsmittel).

Ein nächster Schritt in der Wundversorgung war die Abdeckung von Wunden mit antimikrobiell ausgerüsteten Wundauflagen. Diese Wundauflagen können zum Beispiel durch Behandlung von Trägern wie Geweben, Vliesen, Mull usw. mit Wirkstoffen hergestellt werden. Durch Abdecken von Wunden mit diesen Produkten, die den Wirkstoff nicht abgeben, wird in der Regel aber nur eine Schutzfunktion erreicht. Die Wundauflage bildet lediglich eine Barriere für Mikroorganismen, die am Eindringen in die Wunde von außen gehindert werden, indem sie bei Kontakt mit dem Produkt abgetötet werden. Die Wirkung in der Wunde ist dabei zu vernachlässigen. Es kann also nicht von der Behandlung einer infizierten Wunde gesprochen werden.

Wird der Wirkstoff von antimikrobiell ausgerüsteten Wundauflagen in die Wunde abgegeben, so treten die gleichen Nachteile wie bei Applikation in Reinstform, als Lösung, als Salbe oder in einer anderen Darreichungsform auf. D.h., die Bakterien verbleiben in der Wunde, es ist keine sichere Wundreinigung möglich und es können Störeffekte auftreten.

Neben den beiden oben diskutierten und am weitesten verbreiteten Behandlungsmethoden, nämlich der Applikation von antimikrobiellen Zubereitungen und die Verwendung imprägnierter Wundversorgungsmaterialien, wird auch der Einsatz von hydrophobierten Trägermaterialien beschrieben (EP-B 021 230, EP-B 162 026, EP-A 296 441). In einem hydrophilen Milieu (Wasser, Salzlösung, Wundflüssigkeit) werden hier hydrophobe Bakterien von einer Wundauflage adsorbiert, die durch einen aufwendigen chemischen Prozeß hydrophobiert wurde. Die Bakterien werden dann durch Entfernen der Wundauflage der Wunde entzogen. Entscheidender Nachteil ist dabei, daß im Gegensatz zu den oben aufgeführten, gängigen Behandlungsmethoden Bakterien und Mikroorganismen nicht abgetötet werden. Dieser Nachteil wird noch verstärkt, wenn die behandelte Wunde austrocknet. Dies bedeutet den Verlust des hydrophilen Milieus, das entscheidend zur Wechselwirkung zwischen Wundauflage und Bakterien beiträgt. Die nicht abgetöteten Bakterien und Mikroorganismen lösen sich von der Wundauflage und fallen zurück ins Wundbett.

Aufgabe der Erfindung war es deshalb, eine Wundauflage zu entwickeln, welche die Nachteile des Standes der Technik nicht aufweist und eine verbesserte Behandlung infizierter Wunden und/oder Schutz vor Infektionen ermöglicht. Gelöst wird diese Aufgabe durch eine Wundauflage gemäß Anspruch 1.

Durch die erfindungsgemäße Kombination eines hydrophoben und damit bakterienadsorbierenden Materials und eines antimikrobiellen Wirkstoffs, der nicht in die Wunde abgegeben wird, wird ein neuer Wirkmechanismus mit synergetischem Effekt erzielt.

Die Wundauflage dient als Barriere für Mikroorganismen und sie adsorbiert die Bakterien aus der Wundflüssigkeit. Nach der Adsorption werden diese an der Wundauflage abgetötet und mit dem Entfernen der Auflage werden die abgetöteten Bakterien und unverbrauchter Wirkstoff ebenfalls entfernt. Sie stören damit nicht mehr den Heilungsverlauf.

Geeignete bakterienadsorbierende, hydrophobe Materialien können synthetische oder natürliche oder chemisch modifizierte natürliche Polymere sein wie Polyethylen, Polypropylen, Polyurethan, Polyamid, Polyester, Polyvinylchlorid, Polytetrafluorethylen oder durch kovalente Verknüpfung hydrophiler Substanzen mit hydrophoben Gruppen hergestellte Polymere sein, beispielsweise gemäß EP-B 21 230. Die bakterienadsorbierenden Eigenschaften hydrophober Materialien sind bekannt (vgl. D.F. Gerson et al., Biochim. Biophys. Acta, 602 (1980, 506-510); Y. Fujioka-Hirai. et al., J. of Biochemical Materials Research, Vol. 21, 913-20 (1987); S. Hjerten et al., J. of Chromatography 101 (1974), 281-288; M. Fletcher et al., Appl. and Environmental Mikrobiology, Jan. 1979, 67-72). Die hydrophoben Eigenschaften sind auch einfach durch einen Wassertropfentest nachzuweisen, bei dem das Wasser vom Material abperlt.

Geeignete antimikrobielle Wirkstoffe, unter denen in erster Linie an sich bekannte Substanzen zu verstehen sind, wie z.B. Chlorhexidin oder Phenolderivate wie Thymol und Eugenol oder die in der DE-PS 32 15 134 benannten Chlorphenole oder Chlordiphenylether, zeichnen sich dadurch aus, daß sie fest an der Wundauflage haften, an oder in dieser auf die Mikroorganismen einwirken und nicht oder zumindest nicht merklich in die Wunde abgegeben werden. Dies kann durch physikalische Ein- oder Anlagerung an geeignete Träger erfolgen, z.B. Einlagerung hydrophober Wirkstoffe in hydrophobe Trägermaterialien, oder beispielsweise auch durch kovalente Bindung an diese. Die Wirkstoff/Träger-Systeme sollen sich dadurch auszeichnen, daß auch bei mehreren Extraktionen mit wäßrigen Lösungen oder Wundflüssigkeit ihre antimikrobielle Wirksamkeit erhalten bleibt. Die Wundauflagen sollten den antimikrobiellen Wirkstoff in einer Menge von mindestens 0,001 Gew.-% enthalten, um eine ausreichende Wirksamkeit zu erzielen. Bevorzugt sind es 0,1 - 10%, besonders bevorzugt 0,1 - 2 Gew.-%.

Obwohl die antimikrobiellen Wirkstoffe fest an der Wundauflage haften, sollten sie jedoch dennoch nicht toxisch oder hautreizend sein, um jegliche negative Wirkung beim Patienten auszuschließen.

Die erfindungsgemäßen Wundauflagen, welche in der Regel als mehr oder weniger dünne Flächengebilde ausgestaltet sind, können aus einem hydrophoben Material in Form eines Gewebes, Gewirkes, Netzes, Vlieses, Schaumstoffs oder einer Folie bestehen, welche in oder an sich den antimikrobiellen Wirkstoff enthalten.

In einer weiteren Ausführungsform können sie aus einem Vlies, Gewebe, Gewirke oder Netz mit einerseits hydrophoben Fasern oder Fäden und andererseits den Wirkstoff enthaltenden Faser oder Fäden bestehen. Sie können auch aus gemischten Fäden, die aus hydrophoben Fasern und antimikrobiell wirksamen Fasern bestehen, aufgebaut sein.

Wichtig ist dabei, daß die Wundauflagen insgesamt einen deutlich hydrophoben Charakter aufweisen.

In einer bevorzugten Ausführungsform handelt es sich bei den antimikrobiellen Fasern um Acetatfasern, welche besonders bevorzugt ein chloriertes Phenoxy-Bakterizid enthalten. Derartige Fasern sind als MICROSAFE-Fasern auf dem Markt erhältlich.

Eine weitere Möglichkeit des Aufbaus der Wundauflage besteht darin, daß die hydrophoben Fasern durch ein Bindemittel verbunden sind, welches das antimikrobielle Mittel enthält.

Die Wundauflagen sollten luft- und wasserdampfdurchlässig sein. Da sie an sich jedoch im wesentlichen kein Wundsekret aufnehmen können, kann es sich als vorteilhaft erweisen, sie beispielsweise durch eine Lochung stärker durchlässig zu machen und dann mit einer saugenden Schicht zu hinterlegen. Bei Wunden, die nicht austrocknen dürfen, können sogenannte hydroaktive Wundverbände oder Wundverbände auf Hydrogelbasis über der durchlässigen Wundauflage angebracht werden.

Zur Fixierung der Auflagen können diese beispielsweise auch auf der wundabgewandten Seite mit einem überstehenden, selbstklebend ausgerüsteten Trägermaterial verbunden sein. Die wundzugewandte Seite wird dann üblicherweise mit einer Schutzabdeckung z.B. aus silikonisiertem Papier versehen.

Je nach vorgesehenem Verwendungszweck werden die erfindungsgemäßen antimikrobiellen Wundauflagen - gegebenenfalls wie beschrieben mit anderen Materialien kombiniert - in die gewünschten Formate geschnitten oder gestanzt, eingesiegelt und sterilisiert.

Die nachfolgenden Beispiele sollen die Erfindung beispielsweise erläutern.

### Beispiel 1

Aus einem Faden, der zu 80% aus Polyesterfasern und zu 20% aus Microsafe AM®-Fasern zusammengesetzt ist, wird ein Gewirke hergestellt (ein Trikot-Kettengewirke mit einem Flächengewicht von 170 g/m²). Das Gewirke wird sterilisiert und als Wundauflage unter einem hydroaktiven Wundverband (beispielsweise gemäß WO 94/07935) eingesetzt.

### Beispiel 2

Polypropylenfasern werden mit Microsafe AM®-Fasern der Fa. Hoechst Celanese zu einem Vlies verfestigt (Dicke 0,5 mm, 50 g/m², 90 Gew.-% Polypropylenfasern, 10 Gew.-% Microsafe AM®-Fasern). Das Vlies wird mit einer handelsüblichen Fixierung als Wundauflage verwendet.

### Beispiel 3

Mit einem Dispersionsgießverfahren wird eine hydrophobe Polyurethanfolie auf Basis eines aliphatischen Polyesterurethans hergestellt (Dicke 80 µm). Durch Zusatz einer entsprechenden Menge Thymol (2-Isopropyl-5-methylphenol) zur wäßrigen Dispersion enthält die Folie 0,1 Gew.-% Thymol. Die Folie wird perforiert (Durchmesser der Löcher: 1 mm, perforierte Fläche 15%). Die Folie wird anschließend sterilisiert und als Wundauflage unter einem hydroaktiven Wundverband eingesetzt.

## Patentansprüche

1. Wundauflage, dadurch gekennzeichnet, daß sie aus einem hydrophoben, bakterienadsorbierenden Material besteht, das einen antimikrobiellen Wirkstoff enthält, der nicht in die Wunde abgegeben wird.

2. Wundauflage nach Anspruch 1, dadurch gekennzeichnet, daß das hydrophobe Material in Form eines Gewebes, Gewirkes, Netzes, Vlieses, Schaumstoffs oder einer Folie vorliegt, die den antimikrobiellen Wirkstoff enthalten.

3. Wundauflage nach Anspruch 1, dadurch gekennzeichnet, daß sie aus einem Vlies, Gewebe, Gewirke oder Netz aus hydrophoben Fasern oder Fäden und den antimikrobiellen Wirkstoff enthaltenden Fasern oder Fäden besteht.

4. Wundauflage nach Anspruch 1, dadurch gekennzeichnet, daß sie aus einem Vlies, Gewebe, Gewirke oder Netz besteht mit gemischten Fäden aus hydrophoben Fasern und den antimikrobiellen Wirkstoff enthaltenden Fasern.

5. Wundauflage nach Anspruch 3 oder 4, dadurch gekennzeichnet, daß die den antimikrobiellen Wirkstoff enthaltenden Fasern oder Fäden Acetatfasern oder -fäden sind.

6. Wundauflage nach Anspruch 1, dadurch gekennzeichnet, daß das hydrophobe, bakterienadsorbierende Material ein Bindemittel enthält, welches den antimikrobiellen Wirkstoff enthält und diesen nicht in die Wunde abgibt.

7. Wundauflage nach mindestens einem der vorangehenden Ansprüche, dadurch gekennzeichnet, daß sie wasserdurchlässig ist.

8. Wundauflage nach Anspruch 7, dadurch gekennzeichnet, daß sie mit einer saugenden Auflage hinterlegt ist.

9. Wundauflage nach Anspruch 7, dadurch gekennzeichnet, daß sie mit einer feuchtigkeitsspendenden Auflage hinterlegt ist.

10. Wundauflage nach mindestens einem der vorangehenden Ansprüche, dadurch gekennzeichnet, daß sie auf der wundabgewandten Seite mit einem die Wundauflage überragenden selbstklebend ausgerüsteten Trägermaterial abgedeckt ist.
